# EUROPEAN PATENT SPECIFICATION

(11) **EP 4 143 164 B1**
(45) Date of publication and mention of the grant of the patent: **29.07.2026**
(21) Application number: 21796630.8
(22) Date of filing: 26.04.2021
(51) Int. Cl.: C07D 209/48, A01N 47/04

(54) **PROCESS OF PREPARATION OF A FUNGICIDAL COMPOUND**
VERFAHREN ZUR HERSTELLUNG EINER FUNGIZIDEN VERBINDUNG
PROCÉDÉ DE PRÉPARATION D'UN COMPOSÉ FONGICIDE

(30) Priority: 27.04.2020 IN 202011017964
(43) Date of publication of application: 08.03.2023
(73) Proprietor: UPL Limited, New Delhi 110001 (IN)
(72) Inventor: KINI, Prashant Vasant, New Delhi 110001 (IN); MUKADAM, Vilas Manikant, New Delhi 110001 (IN)
(74) Representative: J A Kemp LLP
(86) International application number: PCT/IN2021/050406
(87) International publication number: WO 2021/220296

(56) References cited:
- US-A- 2 713 058
- US-A- 3 314 969
- US-A- 3 845 122
- US-A- 3 993 693
- US-A- 4 093 651
- GEORGE SOSNOVSKY: "The chemistry of trichloromethanesulfenyl chloride", CHEMICAL REVIEWS, 1 January 1958 (1958-01-01), pages 509 - 540, XP093162886
- PATRIAN BRUNO ET AL: "Carbon Tetrachloride in Folpet Formulations by Headspace GC-MS", 1 January 2012 (2012-01-01), pages 1, XP093138366, Retrieved from the Internet <URL:chrome-extension://efaidnbmnnnibpcajpcglclefindmkaj/https://www.cipac.org/images/pdf/2012/Patrian_CCL4_Handout.pdf> [retrieved on 20240517]
- ANONYMOUS: "Legislation", OFFICIAL JOURNAL OF THE EU, vol. 50, 8 February 2007 (2007-02-08), pages 1 - 40, XP093138290
- ANONYMOUS: "Legislation", OFFICIAL JOURNAL OF THE EU, 9 October 2008 (2008-10-09), pages 1, XP093164099
- ANONYMOUS: "Registration Dossier - ECHA - Captan - Particle size distribution", 13 July 2015 (2015-07-13), XP093164194, Retrieved from the Internet <URL:https://echa.europa.eu/registration-dossier/-/registered-dossier/12775/4/6>
- VELKOSKA-MARKOVSKA, L. ET AL.: "Development and Validation of High- Performance Liquid Chromatography Method for Determination of Some Pesticide Residues in Table Grape", ACTA CHROMATOGRAPHICA, vol. 30, 2018, pages 250 - 254, XP055871797

## Description

### Field of the

The present invention relates to a process for preparation of fungicidal sulfenyl phthalimides compound of formula (I) substantially free from unwanted impurity.

### Background and the prior art:

Sulfenyl phthalimides developed in the 1950s at Standard Oil are one of the oldest groups of fungicides and are effective, safe and persistent. Captan (N-(trichloromethylthio)-3a,4,7,7a-tetrahydrophthalimide, Captafol(N-[1,1,2,2-tetrachloroethylthiol]-4-cyclohexene-1,2-dicarboximide; and Folpet (N-[trichloromethylthio] phthalimide, are the three Sulfenyl phthalimides possessing antifungal activity.

Phthalimide and N-substituted phthalimides are an important class of compounds because they possess important biological activities the identifiable structural features for their activity are as: hydrophobic aryl ring, a hydrogen bonding domain, an electron-donor group, another distal hydrophobic site.

Some synthetic reactions for preparation of phthalimide moiety includes (1) The Mathews' reaction in which a 'dry' hydrolysis of nitriles by phthalic acid or amides by phthalic anhydride takes place and give the corresponding carboxylic acid and phthalimide; (2) reaction of dicarboxylic acids or their corresponding anhydrides with reagents bearing a reactive amino (-NH2) functional group, through a nucleophilic attack of amino group to a anhydride moiety to obtain aromatic or aliphatic cyclic imides and their derivatives; (3) a general synthetic pathway for the synthesis of imides by direct condensation using cyclic anhydrides or their corresponding dicarboxylic acids and form amide which is a simple affordable reagent. This approach has the advantage that this specific reagent can also serve as solvent, especially for aliphatic imides; (4) a preferred method of preparation of sulfenyl phthalimides is reaction of the metal Salt of an imide with perchloromethyl mercaptan (CISCCl₃) in an organic solvent. E.g. synthesis of sulfenyl phthalimide compound, captan is easily possible by Diels-Alder cycloaddition of maleic anhydride with butadiene to the tetrahydrophthalic anhydride , which is then converted with ammonia to the tetrahydrophthalimide. Finally, alkylation of the imide nitrogen atom with perchloromethyl mercaptan delivers Captan.

Folpet and captan are phthalimide based agricultural fungicides that have been in use for over 60 years. The active moiety of each parent chemical is the trichloromethylthio functional group, SCCl₃ which is a toxophore group. The toxophore group, CCl₃-S-, is obtained from trichloromethanesulfenyl chloride, also known as perchloromethyl mercaptan. Both folpet and captan are degraded to the reactive substance thiophosgene, along with relatively stable ring structures. Folpet degrades to phthalimide (PI) and thiophosgene (SCCl₂); captan degrades to 1,2,3,6-tetrahydrophthalimide (THPI) and thiophosgene. PI and THPI are relatively stable.

Captan is a non-systemic fungicide used to control diseases of many fruit, ornamental, and vegetable crops. It is used in agricultural production as well as by the home gardener. Captan can be used to control plant diseases such as black rot, early and late blight, and downy mildew, among others. Captan works by coming into contact with a fungus and interrupting a key process in its life cycle. It can be toxic to many different fungal diseases. Captan is non-systemic, which means it is not expected to move through plants. It is applied to packing and shipping boxes for fruits and vegetables. Captan is used as a preservative for awnings, draperies and leather, as a root dip and seed treatment and is incorporated into paints, wallpaper pastes, plastic and leather goods.

Folpet, a chloroalkylthio compound with broad spectrum protectant fungicide (N-(trichloromethylthio) phth alimide, has been in use for the last several decades.

Folpet is predominantly used in agronomic practice along with other industrial applications today.

During synthesis of sulfenyl phthalimide compounds, manufacturing impurities become important to consider if they are particularly undesirable because of their toxicological, ecotoxicological or environmental properties. Of the several known impurities, carbon tetrachloride (a haloalkane impurity) is of great concern from toxicological point. As per IARC (International Agency for Research on Cancer), carbon tetrachloride induces hepatic cell proliferation and unscheduled DNA synthesis. Carbon tetrachloride has a mutagenic effect and induces aneuploidy in several in-vitro systems. Carbon tetrachloride is possibly carcinogenic to humans (Group 2B). In conclusion, the EU (European Commission) considers carbon tetrachloride as relevant impurity in captan technical and must not exceed maximum levels of 0.1 g/kg in the technical material.

Carbon tetrachloride is toxic to the central nervous system and liver. It is severely hepatotoxic, particularly following ingestion. Liver cell damage is apparently caused by a free radical generated in the process of initial dechlorination. Kidney injury also occurs. Cardiac arrhythmias, progressing to fibrillation, may follow inhalation of high concentrations of carbon tetrachloride or ingestion of the liquid. Carbon tetrachloride impairs the NADPH-dependent oxidative enzymes in liver microsomes by causing irreversible damage to cytochrome P-450. It does not act as a competitive inhibitor. In the liver, carbon tetrachloride produces elevated levels of glutamicoxaloacetic transaminase and aldolase (commonly used in following the clinical course of human patients poisoned by the compound). Centrolobular necrosis of the liver is the lesion most characteristic of poisoning by carbon tetrachloride. The necrosis progresses cell by cell. Electron microscopy reveals vesiculation of the rough endoplasmic reticulum, clumps of tangled, smooth membranes, and vacuolization of the Golgi apparatus. It also reveals loss of polysomes and accumulation of fat. Definite renal tubular lesion, including tubular necrosis and deposition of calcium, have been observed regularly. Mitochondria and not endoplasmic reticulum appear to be the primary subcellular site of carbon tetrachloride toxicity in the kidney.

Carbon tetrachloride also contributes to the destruction of the Earth's ozone layer, which protects us from harmful ultraviolet radiation. Carbon tetrachloride (CCl₄) is an ozone-depleting substance, accounting for about 10% of the chlorine in the troposphere. Under the terms of the Montreal Protocol, its production for dispersive uses was banned under the Montreal Protocol since 2010.

Therefore, it is important to minimize the level of carbon tetrachloride impurity as much as possible while synthesizing sulfenyl phthalimides to avoid its exposure to agricultural workers handling the captan technical as well as to avoid environmental hazards.

US2553770 discloses synthesis of sulfenyl phthalimide compounds. The patent discloses reaction of sulfenyl phthalimide compound in dioxane solvent which is then followed by purification through crystallization in carbon tetrachloride as solvent.

US2553771 discloses synthesis of sulfenyl phthalimide compounds which comprises dissolving an imide of a dicarboxylic acid in an aqueous alkaline solution of an alkali metal compound and reacting the resulting product with perchloromethyl mercaptan. Addition of carbon tetrachloride in both of these patents further contributes to increase the level of carbon tetrachloride impurity in finally synthesized sulfenyl phthalimides.

US2553776 discloses synthesis of captan in which water is used as a solvent in the final step. Also, effect of addition of sodium chloride and potassium chloride to improve the yield of the final product is also disclosed in the said patent. However, patent does not disclose anything about impurity as well as its amount in the final product.

US2713058 discloses an improved method of synthesis of N-trichloromethylthioimides comprising carrying out the reaction of perchloromethyl mercaptan with the alkali metal imide product dissolved in the aqueous media in the presence of a water-immiscible, saturated organic solvent for the perchloromethyl mercaptan. The latter is preferably added to the reaction system dissolved in the organic solvent (saturated C₅-C₉ hydrocarbon) and results in the obtaining of products of substantially increased purity. Although, the N-trichloromethylthioimides obtained according to the invention has more than 95% purity but this patent does not disclose anything about haloalkane impurity and its amount handled during synthesis of N-trichloromethylthioimides.

US3314969 discloses a method for preparing N-trichloromethylthioimide compounds comprises isolating N-polyhaloethylthio compound from the aforesaid reaction mixture as a polar solvent dispersion; contacting said dispersion with an aromatic hydrocarbon solvent of from 6 to 10 carbons at a temperature in the range of about 50 to 100 C. for a time sufficient to dissolve substantially all of the N-polyhaloethylthiocompound; separating the polar solvent phase from the aromatic hydrocarbon solvent phase; cooling the aromatic hydrocarbon phase and isolating therefrom the purified N-poly-haloethylthio compound. US3314969 uses alkali metal salts in the aqueous phase and added the trichloromethylsulfenyl chloride diluted in an aliphatic hydrocarbon solvent to the aqueous solution of the imide. The prior art does not disclose how does carbon tetrachloride been handled in the final product.

Although, attempts have been made to obtain substantially pure sulfenyl phthalimides but it is still required to develop a simple, efficient and cost effective process to obtain sulfenyl phthalimides which has below minimum acceptable limit of haloalkane impurity especially, carbon tetrachloride impurity.

### Objectives of the Invention

An objective of the present invention is to develop fungicidal sulfenyl phthalimide compounds substantially free from unwanted haloalkane impurities.

An objective of the present invention is to develop sulfenyl compound substantially free from haloalkane impurities.

Yet another objective of the present invention is to develop fungicidal sulfenyl phthalimide compounds substantially free from carbon tetrachloride using aromatic hydrocarbon that minimizes haloalkane impurities in the fungicidal sulfenyl phthalimide compounds.

Another object of the invention is to provide a simple process for the preparation of sulfenyl phthalimide compounds in high yield and high purity. The process is simple, easy and convenient to carry out, economical and efficient.

Still another objective of the present invention is to provide a process of preparing fungicidal sulfenyl phthalimide compounds substantially free from haloalkane impurities wherein said process is efficient, simple and cost effective.

Yet another objective of the present invention is to develop a method wherein a high yield of fungicidal sulfenyl phthalimide compounds is obtained.

### Summary of the Invention

In an aspect the present invention relates to prepare a fungicidal sulfenyl phthalimide compound of formula (I) wherein said compound of formula (I) is substantially free from unwanted haloalkane impurities.

In another aspect the present invention relates to prepare a fungicidal sulfenyl phthalimide compound of formula (I) 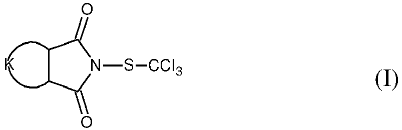 wherein K together with the two contiguous linking carbon atoms, forms a fused 6-membered aromatic ring a or a fused cyclohexene ring, wherein said compound of formula (I) is substantially free from haloalkane impurities.

In another aspect the present invention provides a process for preparation of compound of formula (I) wherein K together with the two contiguous linking carbon atoms, forms a fused cyclohexene ring (that is substantially free from haloalkane impurities wherein said compound of formula (I) is 3a,4,7,7a-Tetrahydro-N-(trichloromethanesulphenyl)phthalimide.

In another aspect the present invention provides a process for preparation of compound of formula (I) wherein K together with the two contiguous linking carbon atoms, forms a fused aromatic ring that is substantially free from haloalkane impurities wherein said compound of formula (I) is N-(Trichloromethanesulphenyl)phthalimide.

In another aspect the present invention provides a process for preparation of compound of formula (II) substantially free from haloalkane impurities used for preparation for compound of formula (I).

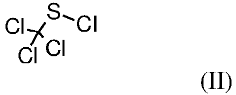

In another aspect of the present invention, a process for the synthesis of fungicidal sulfenyl phthalimide compound of formula (I) which is substantially free from haloalkane impurities comprising :
(a) reacting an organosulphur compound with chlorine to form a sulfenyl compound of formula (II); 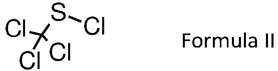
(b) converting said sulfenyl compound of formula (II) to a fungicidal sulfenyl phthalimide compound of formula (I) substantially free from haloalkane impurities.

In another aspect of the present invention, the process for the synthesis of fungicidal sulfenyl phthalimide compound of formula (I) comprising
(a) reacting an organosulphur compound with chlorine to form sulfenyl compound;
(b) isolating pure sulfenyl compound by treating sulfenyl compound with aromatic hydrocarbon to remove haloalkane impurity;
(c) reacting sulfenyl compound of formula (II) with phthalimide or phthalimide derivative to obtain pure sulfenyl phthalimide compound of formula (I);

In another aspect of the present invention, a process for the synthesis of 3a,4,7,7a-Tetrahydro-N-(trichloromethanesulphenyl)phthalimide compound of formula (I) which is substantially free from haloalkane impurity, comprises the steps of:
(a) reacting organosulphur compound with chlorine to form sulfenyl compound;
(b) treating sulfenyl compound of step (a) with aromatic hydrocarbon to remove haloalkane impurity to obtain pure sulfenyl compound of formula (II);
(c) reacting sulfenyl compound of formula (II) with tetrahydropthalimide to obtain pure 3a,4,7,7a-Tetrahydro-N-(trichloromethanesulphenyl)phthalimide compound of formula (I);

In another aspect of the present invention, a process for the synthesis of N-(Trichloromethanesulphenyl)phthalimide compound of formula (I) which is substantially free from haloalkane impurity wherein, said process comprises steps of:
(a) reacting organosulphur compound with chlorine to form sulfenyl compound;
(b) treating sulfenyl compound of step (a) with aromatic hydrocarbon to remove haloalkane impurity to obtain pure sulfenyl compound of formula (II);
(c) reacting sulfenyl compound of formula (II) in an aromatic hydrocarbon with phthalimide to obtain pure N-(Trichloromethanesulphenyl)phthalimide compound of formula (I);

In an aspect of the present invention, the process for the synthesis of sulfenyl compound of formula (II) which is substantially free from haloalkane impurity comprises the steps of:
(a) reacting an organosulphur compound with chlorine to form sulfenyl compound;
(b) treating sulfenyl compound of step (a) with aromatic hydrocarbon to remove haloalkane impurity and obtaining pure sulfenyl compound of formula (II) which is substantially free from haloalkane impurities.

In another aspect of the present invention, a process for the synthesis of fungicidal sulfenyl phthalimide compound of formula (I) which is substantially free from haloalkane impurities wherein, said process comprises: reacting sulfenyl compound of formula (II) in an aromatic hydrocarbon with pthalimide or phthalimde derivative compound and obtaining fungicidal sulfenyl phthalimide compound of formula (I).

### Detailed Description of the invention:

While several embodiments of the present invention have been described and illustrated herein, those of ordinary skill in the art will readily envision a variety of other means and/or structures for performing the functions and/or obtaining the results and/or one or more of the advantages described herein, and each of such variations and/or modifications is deemed to be within the scope of the present invention.

Surprisingly, inventor of the present invention found that a fungicidal sulfenyl phthalimide compounds for formula (I), can be obtained in the substantially pure form with negligible haloalkane impurities, if sulfenyl compound is made by means of chlorination of carbon disulfide, followed by treatment of the obtained crude sulfenyl compound with aromatic hydrocarbons; and reacting sulfenyl compound with phthalimide or phthalimide derivative and further treating with aromatic hydrocarbons to obtain substantially pure sulfenyl phthalimide compound.

Aromatic hydrocarbon treatment used during synthesis of both, sulfenyl compound and sulfenyl phthalimide compounds helps in reducing haloalkane impurities which ultimately leads to substantially pure sulfenyl phthalimide compounds.

Advantageously the present invention has achieved improvements in the production of sulfenyl phthalimide compounds and derivative compounds, for example captan, and folpet via the use of aromatic hydrocarbons which are believed to minimize the formation of the undesirable haloalkane impurities.

Broadly, present invention contemplates a fungicidal sulfenyl phthalimide compound substantially pure from haloalkane impurities and process of preparing fungicidal sulfenyl phthalimide compound substantially pure from haloalkane impurities using aromatic hydrocarbons. The process contemplated by this invention is further explained by the following reaction scheme.

### Step I:

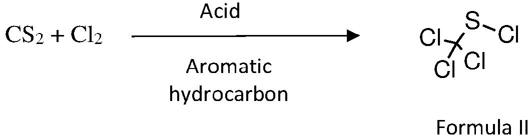

### Step II:

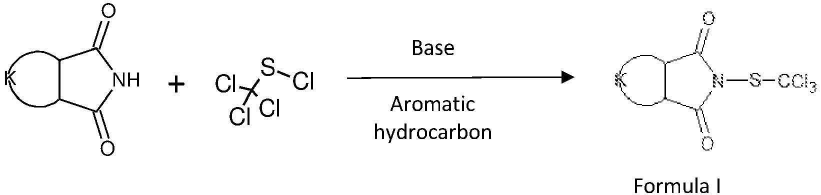

In accordance with the present invention, fungicidal sulfenyl phthalimide compound of formula (I) describe some of the preferred compounds wherein said compound of formula (I) represented as the structure given below is substantially free from haloalkane impurities is, 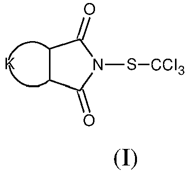 wherein K together with the two contiguous linking carbon atoms, forms a fused 6-membered aromatic ring a or a fused cyclohexene ring.

In an embodiment the present invention provides a compound of formula I wherein K together with the two contiguous linking carbon atoms, forms a fused cyclohexene ring (that is substantially free from haloalkane impurity and process for preparation thereof wherein said compound of formula (I) 3a,4,7,7a-Tetrahydro-N-(trichloromethanesulphenyl)phthalimide and is represented as below:

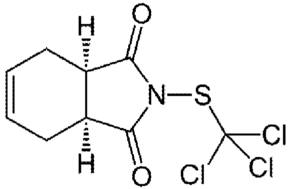

In an embodiment the present invention provides a compound of formula I wherein K together with the two contiguous linking carbon atoms, forms a fused aromatic ring that is substantially free from haloalkane impurities and process for preparation thereof wherein said compound of formula (I) is N-(Trichloromethanesulphenyl)phthalimide represented as below:

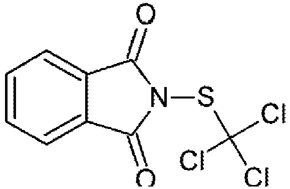

According to an embodiment of the present invention, there is provided a compound of formula (II) substantially free from haloalkane impurity and process for preparation thereof.

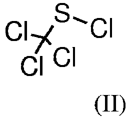

In accordance with the present invention, the compound of formula (II) is also known as Trichloromethane sulfenyl chloride or Perchloromethyl mercaptan (PCMM).

In accordance with the present invention, a fungicidal sulfenyl phthalimide compound of formula (I) is substantially free from haloalkane impurities.

In accordance with the present invention, a fungicidal sulfenyl phthalimide compound of formula (I) substantially free from haloalkane impurities refers to a compound of formula (I) with less than 0.05%, preferably less than 0.01% of haloalkane impurity ( by Gas Chromatography).

In accordance with the present invention, a sulfenyl compound of formula (II) is substantially free from haloalkane impurities.

In accordance with the present invention, a sulfenyl compound of formula (II) substantially free from haloalkane impurities refers to compound of formula (II) with less than 0.10, preferably less than 0.08% of haloalkane impurities ( by Gas Chromatography).

According to an embodiment of the present invention, the haloalkane impurities refers to unwanted impurities belong to the group comprising of carbon tetrachloride and dichloro(chlorosulfanyl)methanesulfonyl chloride or mixture thereof that are generated during the process of synthesis of sulfenyl compound or sulfenyl phthalimide compounds.

Typically, according to a preferred embodiment of the present invention, the haloalkane impurity is carbon tetrachloride.

According to an embodiment of the present invention, the haloalkane impurity present in sulfenyl phthalimide may be any impurities including reaction byproducts, intermediates, starting materials, and solvents.

According to an embodiment of the present invention, haloalkane impurities are capable of being removed azeotropically, such as by vacuum distillation, heat, or low-pressure evaporation.

According to an embodiment of the present invention, haloalkane impurity is preferably removed by distillation.

According to an embodiment of the present invention, a process for the synthesis of fungicidal sulfenyl phthalimide compound of formula (I) which is substantially free from haloalkane impurities wherein, said process comprises steps of:
(a) reacting an organosulphur compound with chlorine to form sulfenyl compound and treating sulfenyl compound with aromatic hydrocarbon to remove haloalkane impurities to obtain pure sulfenyl compound of formula (II) and
(b) reacting sulfenyl compound of formula (II) in an aromatic hydrocarbon with phthalimide or pthalimide derivatives to obtain pure sulfenyl phthalimide compound of formula (I).

In an embodiment, step (a) is carried out in presence of aqueous acidic medium and sulfenyl compound is further treated with aromatic hydrocarbon solvent to obtain pure sulfenyl compound of formula (II) substantially free from haloalkane impurities.

In an embodiment, step b) is carried out in presence of in an aqueous alkaline medium and sulfenyl phthalimide compound is further treated with aromatic hydrocarbon to obtain pure sulfenyl phthalimide compound (I) substantially free from haloalkane impurities.

According to an embodiment of the present invention, a process for the synthesis of fungicidal sulfenyl phthalimide compound of formula (I) which is substantially free from haloalkane impurity wherein, said process comprises steps of:
(a) reacting organosulphur compound with chlorine to obtain crude sulfenyl compound;
(b) treating crude sulfenyl compound of step (a) with aromatic hydrocarbon to remove haloalkane impurity and obtaining sulfenyl compound of formula (II);
(c) reacting sulfenyl compound of formula (II) with phthalimide or pthalimide derivatives to obtain sulfenyl phthalimide compound of formula (I);

According to an embodiment of the present invention, a process for the synthesis of 3a,4,7,7a-Tetrahydro-N-(trichloromethanesulphenyl)phthalimide compound of formula (I) which is substantially free from haloalkane impurities wherein, said process comprises steps of:
(a)reacting organosulphur compound with chlorine to obtain crude sulfenyl compound;
(b)treating crude sulfenyl compound of step (a) with aromatic hydrocarbon to remove haloalkane impurities and obtaining sulfenyl compound of formula (II);
(c)reacting sulfenyl compound of formula (II) in an aromatic hydrocarbon with tetrahydropthalimide to obtain 3a,4,7,7a-Tetrahydro-N-(trichloromethanesulphenyl)phthalimide compound of formula (I).

In step a) the organosulphur compound is carbon disulfide and sulfenyl compound is perchloromethyl mercaptan. Typically, the step a) is carried out at 5 to 10°C. After completion of the reaction of step a) the reaction mass is treated aromatic hydrocarbon solvent. The aromatic hydrocarbon solvent used is selected from the group comprising toluene, chlorobenzene, ethyl benzene, propyl benzene, xylene and the like. The solution thus obtained is then subjected to distillation to remove the unwanted impurities and isolating pure sulfenyl compound which can be directly used for preparation of sulfenyl phthalimide compound of formula (I) without further purification.

In step c) pure sulfenyl compound as obtained in step b) is reacted with tetrahydropthalimide in aqueous basic medium. Typically, the reaction is performed at low temperature preferably at 0 to 10°C, more preferably at 0 to 5°C for 1 to 5 hours preferably for about 1 to 2 hours. The reaction mass is then charged with aromatic hydrocarbon solvent and the mixture is heated to temperature in the range from 50 to 100°C, preferably at 70-80°C and pure sulfenyl phthalimide compound is isolated from the mixture having high purity and high yield as well as reduced unwanted haloalkene impurities preferably to below 0.01%.

According to an embodiment of the present invention, a process for the synthesis of N-(Trichloromethanesulphenyl)phthalimide compound of formula (I) which is substantially free from haloalkane impurities wherein, said process comprises steps of:
(a) reacting organosulphur compound with chlorine to form sulfenyl compound;
(b) treating sulfenyl compound of step (a) with aromatic hydrocarbon to remove haloalkane impurities and obtaining sulfenyl compound of formula (II);
(c) reacting sulfenyl compound of formula (II) with phthalimide to obtain N-(Trichloromethanesulphenyl)phthalimide compound of formula (I).

According to an embodiment of the present invention, a process for the synthesis of sulfenyl compound of formula (II) which is substantially free from haloalkane impurities wherein, said process comprises steps of:
(a) reacting organosulphur compound with chlorine to obtain crude sulfenyl compound;
(b) treating crude sulfenyl compound of step (a) with aromatic hydrocarbon to remove haloalkane impurities and obtaining sulfenyl compound of formula (II) which is substantially free from haloalkane impurities.

In another aspect of the present invention, a process for the synthesis of fungicidal sulfenyl phthalimide compound of formula (I) which is substantially free from haloalkane impurities wherein, said process comprises:
reacting sulfenyl compound of formula (II) with pthalimide or phthalimide derivative and obtaining fungicidal sulfenyl phthalimide compound of formula (I);

According to an embodiment of the present invention, a process for the synthesis of 3a,4,7,7a-Tetrahydro-N-(trichloromethanesulphenyl)phthalimide compound of formula (I) which is substantially free from haloalkane impurities wherein, said process comprises:
reacting sulfenyl compound of formula (II) obtained according to the present process with tetrahydropthalimide and obtaining pure 3a,4,7,7a-Tetrahydro-N-(trichloromethanesulphenyl)phthalimide compound of formula (II) substantially free from haloalkane impurities.

According to an embodiment of the present invention, organosulphur compound is selected from the group comprising of carbon disulfide, carbonyl sulphide, thiophosgene and the like.

According to preferred embodiment of the present invention, organosulphur compound carbon disulfide.

According to an embodiment of the present invention, acid medium utilized in step (a) to form sulfenyl compound of formula (II) is selected from the group comprising of hydrochloric acid (HCl), phosphoric acid, sulfuric acid.

According to preferred embodiment of the present invention, acid medium utilized in step (a) to form sulfenyl compound of formula (II) is hydrochloric acid (HCl).

According to an embodiment of the present invention, alkaline medium utilized in step (b) to form sulfenyl phthalimide compound of formula (I) is selected from the group comprising of sodium hydroxide (NaOH), potassium hydroxide (KOH), Lithium hydroxide (LiOH), potassium carbonate (K₂CO₃) and the like.

According to preferred embodiment of the present invention, alkaline medium utilized in step (b) to form sulfenyl phthalimide compound of formula (I) is sodium hydroxide (NaOH).

According to an embodiment of the present invention, aromatic hydrocarbon used for synthesis of sulfenyl phthalimide compound of formula (I) and sulfenyl compound of formula (II) are selected from the group comprising of toluene, chlorobenzene, ethyl benzene, propyl benzene, xylene and the like.

According to preferred embodiment of the present invention, aromatic hydrocarbon used for synthesis of sulfenyl phthalimide compound of formula (I) and sulfenyl compound of formula (II) is toluene.

Accordingly, a fungicidal sulfenyl phthalimide compound of formula (I), obtained by the present process as described above is substantially free from haloalkane impurities.

Preferably, 3a,4,7,7a-Tetrahydro-N- (trichloromethanesulphenyl)phthalimide obtained by the present process is substantially free from haloalkane impurities.

Preferably, N-(Trichloromethanesulphenyl) phthalimide obtained by the present process is substantially free from haloalkane impurities.

In an embodiment, the process according to the present invention provides fungicidal sulfenyl phthalimide having particle size distribution D₁₀ of less than about 23.101 microns.

In an embodiment, the process according to the present invention provides fungicidal sulfenyl phthalimide having particle size distribution D₅₀ of less than about 72.223 microns.

In an embodiment, the process according to the present invention provides fungicidal sulfenyl phthalimide having particle size distribution D₉₀ of less than about 172.728 microns.

According to the invention the compound of formula (1) is obtained in high yield and has high purity of more than 98.5 %. The present process is simple, easy and convenient to carry out, efficient, economical and also industrially and commercially viable.

According to an embodiment of the present invention, fungicidal sulfenyl phthalimide compound may be used in any kind of solid or liquid formulation meant for agrochemical application along with other optional components including but not limited to surfactants, dispersing agents; wetting agents; antifoaming agents; antimicrobial agents; antioxidants; buffers; dyes; perfumes; stabilizing agents; and water-soluble salts.

According to an embodiment of the present invention, fungicidal sulfenyl phthalimide compound may also be mixed with other agrochemically acceptable ingredients, for example fertilizers such as ammonium nitrate, urea, potash, and superphosphate; phytotoxicants and plant growth regulators; safeners; and pesticides.

According to an embodiment present invention provides a fungicide composition comprising a fungicidal sulfenyl phthalimide compound of formula (I) substantially free from haloalkane impurities prepared according to present process. In another embodiment the present invention provides a method of treating fungal infection by applying to the locus a fungicidal sulfenyl phthalimide compound of formula (I) substantially free from haloalkane impurities prepared according to present process. It will be apparent to those skilled in the art that various modifications and variations can be made in the present invention without departing from the scope of the invention. Other embodiments of the invention will be apparent to those skilled in the art from consideration of the specification and practice of the invention disclosed herein. It is intended that the specification and examples be considered as exemplary only, with a true scope. The process of preparing sulfenyl phthalimide compound is illustrated in the following examples:

### EXAMPLES

### Example 1:

### Synthesis of 3a,4,7,7a-Tetrahydro-N-(trichloromethanesulphenyl) phthalimide

**Step 1:** In 1664g water, 974g (30%) aqueous HCl solution and 380g carbon disulfide was charged at 5-10°C and chlorine gas was purged for 22-24 hrs at same temperature. After completion of reaction, layers were separated, organic mass was collected and toluene (210 gm) was added and further organic layer was washed two times with 250 gm water. 905 g crude Perchloromethyl Mercaptan (PCMM) toluene solution was obtained. Distillation was performed of crude PCMM toluene solution on 3 feet packed column to remove low boiler and haloalkane impurity, carbon tetrachloride (CTC) along with toluene under vacuum. The PCMM toluene solution thus obtained was further taken for step 2. Final sulfenyl compound (the PCMM) specification: Residual PCMM: (778.0 g). Yield: 77.0%, Purity: 92.61% with CTC-0.07 % (by GC).

**Step 2:** In 400 g water, 44.60 g (48%) NaOH solution was charged and then cooled to 10-15°C. To the above solution, 81.38 g tetrahydrophthalimide was slowly added in 10-15 min span and further stirred for 45 min at same temperature clear solution was observed and then cooled to 0-2°C to obtain reaction mass. Separately, 100.4 g PCMM solution obtained in step 1 was added to above reaction mass during addition precipitation was observed and stirred for 2.0 hrs at same temperature. In work up, toluene (323 g) was charged and reaction mass was heated to 75-80°C under stirring for 30 min and cooled to 20-25°C, filtered and the washed with toluene (85 g) and hot water (98 g) two times to finally give an isolated dry solid of 133.4 g. Final sulfenyl phthalimide compound specification: Yield: 88.78%, Purity: 98.70 % with 0.0092 % CTC.

### Example 2:

### Synthesis of 3a,4,7,7a-Tetrahydro-N-(trichloromethanesulphenyl) phthalimide

**Step-I:** In 323.6 g water, 188.8g (30%) aqueous HCl solution and carbon disulfide (73.6 g) was charged at 5-10°C and chlorine gas was purged for 22-24 hrs at the same temperature. After completion of reaction, layers were separated. chlorobenzene (48.4 gm) was added to organic mass and washed two times with 48.4 gm water. 192.0g crude Perchloromethyl Mercaptan (PCMM) chlorobenzene solution was obtained. Distillation was performed of crude PCMM chlorobenzene solution on 3 feet packed column to remove low boiler and haloalkane impurity, carbon tetrachloride (CCl₄) along with chlorobenzene under vacuum. The PCMM chlorobenzene solution thus obtained was further taken for step 2. Final sulfenyl compound (the PCMM) specification: Residual PCMM: (147.2 g). Yield: 72.0 %, Purity: 88.10 % with, CTC-0.06%.

**Step 2:** In 302.4 g water, 33.72g (48%) NaOH solution was charged and then cooled to 10-15°C. To the above solution, 61.3g tetrahydrophthalimide was slowly added in 10-15 min span and further stirred for 45 min at same temperature clear solution was observed and then cooled to 0-2°C to obtain reaction mass. Separately, 79.8 g PCMM solution obtained in step 1 was added to above reaction mass during addition precipitation was observed and stirred for 2.0 hrs at same temperature. In work up, chlorobenzene (244 g) was charged and reaction mass was heated to 75-80°C under stirring for 30 min and cooled to 20-25°C, filtered and the washed with chlorobenzene (64.2 g) and hot water (74.1 g) two times to finally give an isolated dry solid of 91.5 g. Final sulfenyl phthalimide compound specification: Yield: 80.54%, Purity: 97.80 % with CTC-0.008%.

### Example 3:

### Synthesis of 3a,4,7,7a-Tetrahydro-N-(trichloromethanesulphenyl) phthalimide

**Step-I:** In 323.6g water, 188.8g (30%) aqueous HCl solution and carbon disulfide (73.6 g) was charged at 5-10°C and chlorine gas was purged for 22-24 hrs at the same temperature. After completion of reaction, layers were separated. Xylene (48.4 gm) was added to organic mass and washed two times with 48.4 gm water. 194.0g crude Perchloromethyl Mercaptan (PCMM) xylene solution was obtained. Distillation was performed of crude PCMM xylene solution on 3 feet packed column to remove low boiler and haloalkane impurity, carbon tetrachloride (CCl₄) along with xylene under vacuum. The PCMM xylene solution thus obtained was further taken for step 2. Final sulfenyl compound (the PCMM) specification: Residual PCMM: (143.0 g). Yield: 70.40%, Purity: 88.70% with CTC-0.07%.

**Step 2:** In 302.4 g water, 33.72g (48%) NaOH solution was charged and then cooled to 10-15°C. To the above solution, 61.3g tetrahydrophthalimide was slowly added in 10-15 min span and further stirred for 45 min at same temperature clear solution was observed and then cooled to 0-2°C to obtain reaction mass. Separately, 79.3g PCMM solution obtained in step-1 was added to above reaction mass during addition precipitation was observed and stirred for 2.0 hrs at same temperature. In work up, xylene (244 g) was charged and reaction mass was heated to 75-80°C under stirring for 30 min and cooled to 20-25°C, filtered and the washed with xylene (64.2 g) and hot water (74.1 g) two times to finally give an isolated dry solid of 88.0 g. Final sulfenyl phthalimide compound specification: Yield: 77.46%, Purity: 97.71 % with CTC 0.0084% .

### Example 4:

### Synthesis of 3a,4,7,7a-Tetrahydro-N-(trichloromethanesulphenyl) phthalimide

**Step-I:** In 323.6g water, 188.8g (30%) aqueous HCl solution and carbon disulfide (73.6 g) was charged at 5-10°C and chlorine gas was purged for 22-24 hrs at the same temperature. After completion of reaction, layers were separated. Ethyl benzene (48.4 gm) was added to organic mass and washed two times with 48.4 gm water. 190.0g crude Perchloromethyl Mercaptan (PCMM) ethyl benzene solution was obtained. Distillation was performed of crude PCMM ethyl benzene solution on 3 feet packed column to remove low boiler and haloalkane impurity, carbon tetrachloride (CCl₄) along with ethyl benzene under vacuum. The PCMM ethyl benzene solution thus obtained was further taken for step 2. Final sulfenyl compound (the PCMM) specification: Residual PCMM: (146.3 g). Yield: 71.80%, Purity: 88.40% w/w with CTC-0.06%.

**Step 2:** In 302.4 g water, 33.72g (48%) NaOH solution was charged and then cooled to 10-15°C. To the above solution, 61.3g tetrahydrophthalimide was slowly added in 10-15 min span and further stirred for 45 min at same temperature clear solution was observed and then cooled to 0-2°C to obtain reaction mass. Separately, 79.60 g PCMM solution obtained in step 1was added to above reaction mass during addition precipitation was observed and stirred for 2.0 hrs at same temperature. In work up, ethyl benzene (244 g) was charged and reaction mass was heated to 75-80°C under stirring for 30 min and cooled to 20-25°C, filtered and the washed with ethyl benzene (64.2 g) and hot water (74.1 g) two times to finally give an isolated dry solid of 95.0 g. Final sulfenyl phthalimide compound specification: Yield: 83.6%, Purity: 98.61 % with CTC-0.0088%.

### Example 5: Synthesis of Perchloromethylmercaptan (PCMM)

In 1664g water, 974g (30%) aqueous HCl solution and 380g carbon disulfide was charged at 5-10°C and chlorine gas was purged for 22-24 hrs at same temperature. After completion of reaction, layers were separated, organic mass was collected and toluene (210 gm) was added and further organic layer was washed two times with 250 gm water. 905 g crude Perchloromethyl Mercaptan (PCMM) toluene solution was obtained. Distillation was performed of crude PCMM toluene solution on 3 feet packed column to remove low boiler and haloalkane impurity, carbon tetrachloride (CCl₄) along with toluene under vacuum. Final sulfenyl compound (the PCMM) specification: Residual PCMM: (778.0 g). Yield: 77.0%, Purity: 92.61% with CTC-0.07 %.

### Example 6: Synthesis of N-(Trichloromethanesulphenyl)phthalimide

**Step 1:** In 1664g water, 974g (30%) aqueous HCl solution and 380g carbon disulfide was charged at 5-10°C and chlorine gas was purged for 22-24 hrs at same temperature. After completion of reaction, layers were separated, organic mass was collected and toluene (210 gm) was added and further organic layer was washed two times with 250 gm water. 905 g crude Perchloromethyl Mercaptan (PCMM) toluene solution was obtained. Distillation was performed of crude PCMM toluene solution on 3 feet packed column to remove low boiler and haloalkane impurity, carbon tetrachloride (CCl₄) along with toluene under vacuum. The PCMM toluene solution thus obtained was further taken for step 2. Final sulfenyl compound (the PCMM) specification: Residual PCMM: (778.0 g). Yield: 77.0%, Purity: 92.61% with CTC-0.07 %.

**Step 2:** In 200 g water, 24 g (48%) NaOH solution was charged and then cooled to 10-15°C. To the above solution, 40.3g phthalimide was slowly added in 10-15 min span and further stirred for 45 min at same temperature clear solution was observed and then cooled to 0-2°C to obtain reaction mass. Separately, 53.98g PCMM solution obtained in step 1 was added to reaction mass during addition precipitation was observed and stir it for 2.0 hrs at same temperature. In work up, toluene (174 g) was charged and reaction mass was heated to 75-80°C under stirring for 30 min and cooled to 20-25°C, filtered and the washed with toluene (45.6 g) and hot water (52.6 g) two times to finally give an isolated dry solid of 55.8 g. Final sulfenyl phthalimide compound specification: Yield: 70 %, Purity: 96.0 % .

Therefore, the fungicidal sulfenyl phthalimide compound substantially free from haloalkane impurity was successfully prepared using a process according to the present invention. Treatment of crude sulfenyl compound with aromatic hydrocarbon results into sulfenyl compound with lesser haloalkane impurity and the resulting sulfenyl compound further utilized for synthesizing sulfenyl phthalimide compound substantially free from haloalkane impurity. It is to be understood that the invention is not to be limited to the details of the above embodiments, which are described by way of example only.

## Claims

1. A process for preparation of fungicidal sulfenyl phthalimide compound of formula (I) 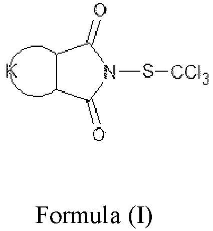 wherein K together with the two contiguous linking carbon atoms, forms a 6-membered fused aromatic ring or a fused cyclohexene ring;
said process comprising:
(a) reacting an organosulphur compound with chlorine in the presence of an aqueous acidic medium to form a sulfenyl compound of formula (II), wherein sulfenyl compound is further treated with an aromatic hydrocarbon to obtain sulfenyl compound of formula (II) substantially free from haloalkane impurities; 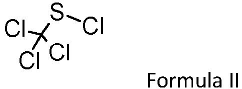
(b) converting said sulfenyl compound of formula (II) to a fungicidal sulfenyl phthalimide compound of formula (I) substantially free from haloalkane impurities, wherein step b) comprises reacting the sulfenyl compound of formula (II) with a phthalimide or phthalimide derivative compound in the presence of an aqueous alkaline medium, further wherein the sulfenyl phtalimide compound is further treated with an aromatic hydrocarbon; 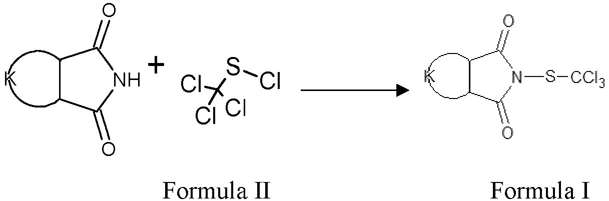 and wherein said haloalkane impurity is carbon tetrachloride,.

2. The process as claimed in claim 1 wherein the reaction in step (a) is carried out at temperature in the range from 5 to 20°C.

3. The process as claimed in claim 1 wherein the reaction in step b) is carried out at temperature in the range from 0 to 10°C.

4. The process as claimed in claim 1, wherein the treatment with the aromatic hydrocarbon in step b) involves charging the reaction mixture with the aromatic hydrocarbon and heating the resulting mixture to a temperature in the range of from 50 to 100 °C, preferably from 70 to 80°C.

5. The process as claimed in claim 1 wherein said organosulphur compound is selected from carbon disulfide, carbonyl sulphide and thiophosgene.

6. The process as claimed in claim 1 wherein acid used for acidic solution is selected from hydrochloric acid, phosphoric acid and sulfuric acid.

7. The process as claimed in claim 1 wherein alkali used for alkaline medium is selected from sodium hydroxide, potassium hydroxide, lithium hydroxide and potassium carbonate.

8. The process as claimed in claim 1 wherein said aromatic hydrocarbon is selected from toluene, chlorobenzene, ethyl benzene, propyl benzene and xylene.

9. The process as claimed in claim 1 comprising preparation of fungicidal sulfenyl phthalimide compound of formula (I) by
a) reacting carbon disulphide with chlorine in an aqueous acidic solution to obtain perchloromethyl Mercaptan and treating perchloromethyl Mercaptan with an aromatic hydrocarbon to obtain perchloromethyl mercaptan which is substantially free from haloalkane impurities; and 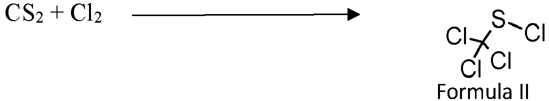
b) reacting compound of formula (II) in an aromatic hydrocarbon solvent with phthalimide or phthalimide derivative compound in an aqueous alkaline medium to obtain sulfenyl phthalimide compound of formula (I) substantially free from haloalkane impurities, 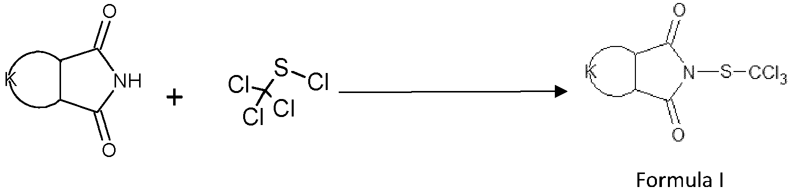
wherein said compound of formula (II) and said compound of formula of (I) is substantially free from carbon tetrachloride impurity.

10. The process as claimed in claim 9, wherein said carbon tetrachloride impurity in the compound of Formula (I) is reduced to less than 0.05% in the process.

11. The process as claimed in claim 1 wherein said compound of formula (II) and said compound of formula of (I) obtained is about 98% pure.

12. The process as claimed in claim 1 wherein said sulfenyl phthalimide compound of formula (I) is selected from 3a,4,7,7a-Tetrahydro-N-(trichloromethanesulphenyl)phthalimide or N-(Trichloromethanesulphenyl)phthalimide.

13. The process according to claim 12, wherein the compound of formula (I) is 3a,4,7,7a-Tetrahydro-N- (trichloromethanesulphenyl)phthalimide.

14. The process as claimed in claim 12 wherein said 3a,4,7,7a-Tetrahydro-N-(trichloromethanesulphenyl)phthalimide compound is prepared by
(a) reacting organosulphur compound with chlorine in presence an aqueous acidic solution to obtain crude perchloromethyl mercaptan;
(b) isolating perchloromethyl mercaptan substantially free from haloalkane impurities by treating crude perchloromethyl mercaptan with aromatic hydrocarbon; and
(c) reacting perchloromethyl mercaptan with tetrahydropthalimide in presence of aqueous alkaline medium to obtain 3a,4,7,7a-Tetrahydro-N-(trichloromethanesulphenyl)phthalimide compound substantially free from haloalkane impurities.

15. The process as claimed in claim 12, wherein said N-(Trichloromethanesulphenyl)phthalimide compound is prepared by
(a) reacting carbon disulfide with chlorine in an aqueous acidic solution to obtain crude perchloromethyl mercaptan;
(b) isolating perchloromethyl mercaptan substantially free from haloalkane impurities by treating crude perchloromethyl mercaptan obtained in step (a) with aromatic hydrocarbon and
(c) reacting perchloromethyl mercaptan in aromatic hydrocarbon with phthalimide compound in an aqueous alkaline medium to obtain N-(Trichloromethanesulphenyl)phthalimide compound substantially free from haloalkane impurities.

16. A process for preparation of perchloromethyl mercaptan of formula (II) comprising
(a) reacting organosulphur compound with chlorine in an aqueous acidic solution to obtain crude sulfenyl compound; and
(b) treating crude sulfenyl compound with aromatic hydrocarbon and isolating perchloromethyl mercaptan of formula (II) substantially free from haloalkane impurities.

17. The process as claimed in claim 16 comprising reacting carbon disulfide with chlorine to obtain perchloromethyl mercaptan and treating perchloromethyl mercaptan with toluene to obtain perchloromethyl mercaptan substantially free from haloalkane impurities.

## Patentansprüche

1. Verfahren zur Herstellung einer fungiziden Sulfenylphthalimid-Verbindung der Formel (I) 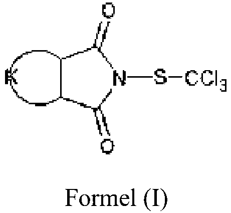
wobei K zusammen mit den beiden benachbarten verbindenden Kohlenstoffatomen einen 6-gliedrigen kondensierten aromatischen Ring oder einen kondensierten Cyclohexenring bildet;
wobei das Verfahren umfasst:
(a) Umsetzen einer Organoschwefel-Verbindung mit Chlor in Gegenwart eines wässrigen sauren Mediums, um eine Sulfenyl-Verbindung der Formel (II) zu bilden, wobei die Sulfenyl-Verbindung ferner mit einem aromatischen Kohlenwasserstoff behandelt wird, um eine Sulfenyl-Verbindung der Formel (II) zu erhalten, die im Wesentlichen frei von Halogenalkan-Verunreinigungen ist; 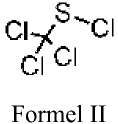
(b) Umwandeln der Sulfenyl-Verbindung der Formel (II) in eine fungizide Sulfenylphthalimid-Verbindung der Formel (I), die im Wesentlichen frei von Halogenalkan-Verunreinigungen ist, wobei Schritt b) das Umsetzen der Sulfenyl-Verbindung der Formel (II) mit einer Phthalimid- oder Phthalimidderivat-Verbindung in Gegenwart eines wässrigen alkalischen Mediums umfasst, ferner wobei die Sulfenylphthalimid-Verbindung ferner mit einem aromatischen Kohlenwasserstoff behandelt wird; 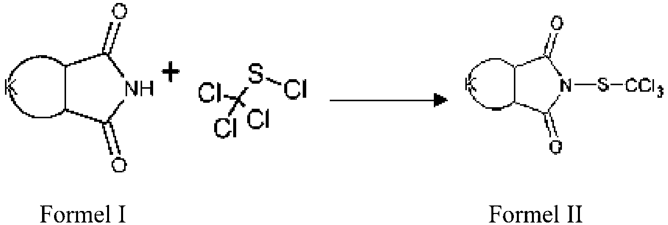
und wobei die Halogenalkan-Verunreinigung Tetrachlorkohlenstoff ist.

2. Verfahren nach Anspruch 1, wobei die Reaktion in Schritt (a) bei einer Temperatur im Bereich von 5 bis 20 °C durchgeführt wird.

3. Verfahren nach Anspruch 1, wobei die Reaktion in Schritt (b) bei einer Temperatur im Bereich von 0 bis 10 °C durchgeführt wird.

4. Verfahren nach Anspruch 1, wobei die Behandlung mit dem aromatischen Kohlenwasserstoff in Schritt b) das Einbringen des aromatischen Kohlenwasserstoffs in das Reaktionsgemisch und das Erhitzen des resultierenden Gemischs auf eine Temperatur im Bereich von 50 bis 100 °C, vorzugsweise von 70 bis 80 °C, umfasst.

5. Verfahren nach Anspruch 1, wobei die Organoschwefel-Verbindung ausgewählt ist aus Schwefelkohlenstoff, Carbonylsulfid und Thiophosgen.

6. Verfahren nach Anspruch 1, wobei die für die saure Lösung verwendete Säure ausgewählt ist aus Salzsäure, Phosphorsäure und Schwefelsäure.

7. Verfahren nach Anspruch 1, wobei das für das alkalische Medium verwendete Alkali aus Natriumhydroxid, Kaliumhydroxid, Lithiumhydroxid und Kaliumcarbonat ausgewählt ist.

8. Verfahren nach Anspruch 1, wobei der aromatische Kohlenwasserstoff aus Toluol, Chlorbenzol, Ethylbenzol, Propylbenzol und Xylol ausgewählt ist.

9. Verfahren nach Anspruch 1, das die Herstellung einer fungiziden Sulfenylphthalimid-Verbindung der Formel (I) umfasst durch
(a) Umsetzen von Schwefelkohlenstoff mit Chlor in einer wässrigen sauren Lösung, um Perchlormethylmercaptan zu erhalten, und Behandeln von Perchloromethylmercaptan mit einem aromatischen Kohlenwasserstoff, um Perchloromethylmercaptan zu erhalten, das im Wesentlichen frei von Halogenalkan-Verunreinigungen ist; und 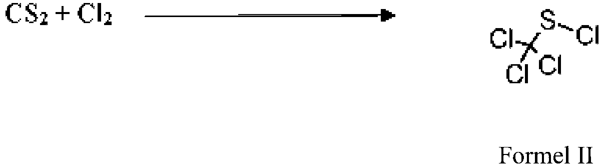 (b) Umsetzen einer Verbindung der Formel (II) in einem aromatischen Kohlenwasserstoff-Lösungsmittel mit Phthalimid oder einer Phthalimidderivat-Verbindung in einem wässrigen alkalischen Medium, um eine Sulfenylphthalimid-Verbindung der Formel (I) zu erhalten, die im Wesentlichen frei von Halogenalkan-Verunreinigungen ist, 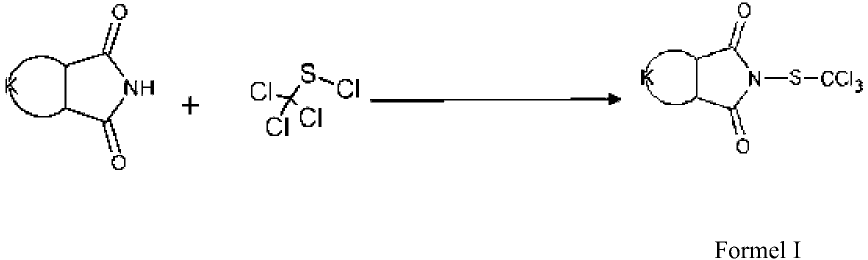 wobei die Verbindung der Formel (II) und die Verbindung der Formel (I) im Wesentlichen frei von Tetrachlorkohlenstoff-Verunreinigungen sind.

10. Verfahren nach Anspruch 9, wobei die Tetrachlorkohlenstoff-Verunreinigung in der Verbindung der Formel (I) in dem Verfahren auf weniger als 0,05 % reduziert wird.

11. Verfahren nach Anspruch 1, wobei die erhaltene Verbindung der Formel (II) und die Verbindung der Formel (I) eine Reinheit von etwa 98 % aufweisen.

12. Verfahren nach Anspruch 1, wobei die Sulfenylphthalimid-Verbindung der Formel (I) aus 3a,4,7,7a-Tetrahydro-N-(trichlormethansulfenyl)phthalimid oder N-(Trichlormethansulfenyl)phthalimid ausgewählt ist.

13. Verfahren nach Anspruch 12, wobei die Verbindung der Formel (I) 3a,4,7,7a-Tetrahydro-N-(trichlormethansulfenyl)phthalimid ist.

14. Verfahren nach Anspruch 12, wobei die 3a,4,7,7a-Tetrahydro-N-(trichlormethansulfonyl)phthalimid-Verbindung hergestellt wird durch
(a) Umsetzen einer Organoschwefel-Verbindung mit Chlor in Gegenwart einer wässrigen sauren Lösung, um rohes Perchlormethylmercaptan zu erhalten;
(b) Isolieren von Perchloromethylmercaptan, das im Wesentlichen frei von Halogenalkan-Verunreinigungen ist, durch Behandeln des rohen Perchloromethylmercaptans mit einem aromatischen Kohlenwasserstoff; und
(c) Umsetzen von Perchloromethylmercaptan mit Tetrahydrophthalimid in Gegenwart eines wässrigen alkalischen Mediums, um eine 3a,4,7,7a-Tetrahydro-N-(trichlormethansulfenyl)phthalimid-Verbindung zu erhalten, die im Wesentlichen frei von Halogenalkan-Verunreinigungen ist.

15. Verfahren nach Anspruch 12, wobei die N-(Trichlormethansulfonylphenyl)phthalimid-Verbindung hergestellt wird durch
(a) Umsetzen von Schwefelkohlenstoff mit Chlor in einer wässrigen sauren Lösung, um rohes Perchloromethylmercaptan zu erhalten;
(b) Isolieren von Perchloromethylmercaptan, das im Wesentlichen frei von Halogenalkan-Verunreinigungen ist, durch Behandeln des in Schritt (a) erhaltenen rohen Perchloromethylmercaptans mit einem aromatischen Kohlenwasserstoff und
(c) Umsetzen des in aromatischem Kohlenwasserstoff befindlichen Perchloromethylmercaptans mit der Phthalimid-Verbindung in einem wässrigen alkalischen Medium, um eine N-(Trichlormethansulphenyl)phthalimid-Verbindung zu erhalten, die im Wesentlichen frei von Halogenalkan-Verunreinigungen ist.

16. Verfahren zur Herstellung von Perchloromethylmercaptan der Formel (II), das umfasst
(a) Umsetzen einer Organoschwefel-Verbindung mit Chlor in einer wässrigen sauren Lösung, um eine rohe Sulfenyl-Verbindung zu erhalten; und
(b) Behandeln der rohen Sulfenyl-Verbindung mit einem aromatischen Kohlenwasserstoff und Isolieren von Perchloromethylmercaptan der Formel (II), das im Wesentlichen frei von Halogenalkan-Verunreinigungen ist.

17. Verfahren nach Anspruch 16, das umfasst: Umsetzen von Schwefelkohlenstoff mit Chlor, um Perchloromethylmercaptan zu erhalten, und Behandeln des Perchloromethylmercaptans mit Toluol, um Perchloromethylmercaptan zu erhalten, das im Wesentlichen frei von Halogenalkan-Verunreinigungen ist.

## Revendications

1. Procédé de préparation d'un composé de phtalimide de sulfényle fongicide de formule (I) 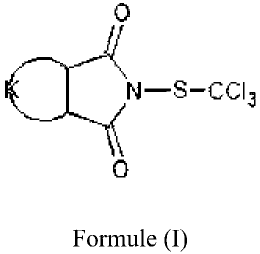 où K, conjointement avec les deux atomes de carbone de liaison contigus, forme un cycle aromatique fusionné à 6 chaînons ou un cycle cyclohexène fusionné, ledit procédé comprenant :
(a) la réaction d'un composé organosulfuré avec du chlore en présence d'un milieu aqueux acide pour former un composé de sulfényle de formule (II), le composé de sulfényle étant ensuite traité avec un hydrocarbure aromatique pour obtenir un composé de sulfényle de formule (II) sensiblement exempt d'impuretés halogénoalcane, 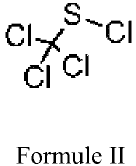
(b) la conversion dudit composé de sulfényle de formule (II) en un composé de phtalimide de sulfényle fongicide de formule (I) sensiblement exempt d'impuretés halogénoalcane, l'étape b) comprenant la réaction du composé de sulfényle de formule (II) avec un composé de phtalimide ou un composé de dérivé de phtalimide en présence d'un milieu alcalin aqueux, le composé de phtalimide de sulfényle étant en outre traité avec un hydrocarbure aromatique, 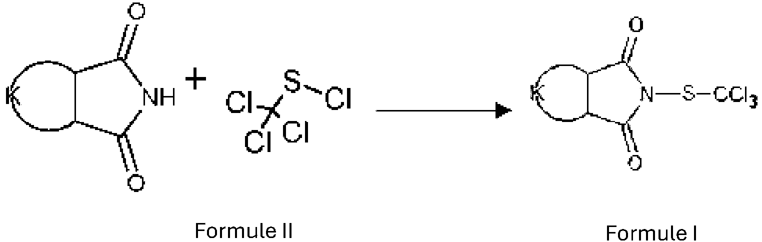 et ladite impureté halogénoalcane étant le tétrachlorure de carbone.

2. Procédé selon la revendication 1, dans lequel la réaction à l'étape (a) est réalisée à une température comprise dans la plage de 5 à 20 °C.

3. Procédé selon la revendication 1, dans lequel la réaction à l'étape (b) est réalisée à une température comprise dans la plage de 0 à 10 °C.

4. Procédé selon la revendication 1, dans lequel le traitement avec l'hydrocarbure aromatique à l'étape b) consiste à introduire l'hydrocarbure aromatique dans le mélange de réaction et à chauffer le mélange résultant à une température comprise dans la plage de 50 à 100 °C, de préférence de 70 à 80 °C.

5. Procédé selon la revendication 1, dans lequel ledit composé organosulfuré est choisi parmi le disulfure de carbone, le sulfure de carbonyle et le thiophosgène.

6. Procédé selon la revendication 1, dans lequel l'acide utilisé pour la solution acide est choisi parmi l'acide chlorhydrique, l'acide phosphorique et l'acide sulfurique.

7. Procédé selon la revendication 1, dans lequel un alcali utilisé pour le milieu alcalin est choisi parmi l'hydroxyde de sodium, l'hydroxyde de potassium, l'hydroxyde de lithium et le carbonate de potassium.

8. Procédé selon la revendication 1, dans lequel ledit hydrocarbure aromatique est choisi parmi le toluène, le chlorobenzène, l'éthylbenzène, le propylbenzène et le xylène.

9. Procédé selon la revendication 1, comprenant la préparation d'un composé de phtalimide de sulfényle fongicide de formule (I) par
a) réaction de disulfure de carbone avec du chlore dans une solution acide aqueuse pour obtenir du mercaptan méthylique perchloré, et traitement du mercaptan méthylique perchloré avec un hydrocarbure aromatique pour obtenir du mercaptan méthylique perchloré qui est sensiblement exempt d'impuretés halogénoalcane, et 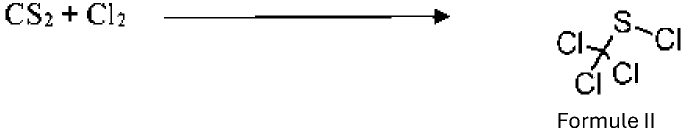
b) réaction du composé de formule (II) dans un solvant hydrocarboné aromatique avec un composé de phtalimide ou un composé de dérivé de phtalimide dans un milieu alcalin aqueux pour obtenir un composé de phtalimide de sulfényle de formule (I) sensiblement exempt d'impuretés halogénoalcane, 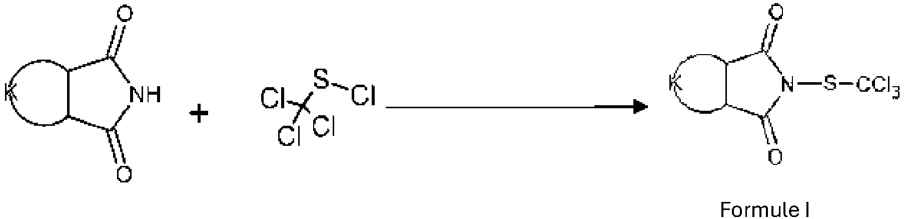
ledit composé de formule (II) et ledit composé de formule (I) étant sensiblement exempts d'impureté tétrachlorure de carbone.

10. Procédé selon la revendication 9, dans lequel ladite impureté tétrachlorure de carbone dans le composé de formule (I) est réduite à moins de 0,05 % au cours du procédé.

11. Procédé selon la revendication 1, dans lequel ledit composé de formule (II) et ledit composé de formule (I) obtenus présentent une pureté d'environ 98 %.

12. Procédé selon la revendication 1, dans lequel ledit composé de phtalimide de sulfényle de formule (I) est choisi parmi le 3a,4,7,7a-tétrahydro-N-(trichlorométhanesulfényl)phtalimide et le N-(trichlorométhanesulfényl)phtalimide.

13. Procédé selon la revendication 12, dans lequel le composé de formule (I) est le 3a,4,7,7a-tétrahydro-N-(trichlorométhanesulfényl)phtalimide.

14. Procédé selon la revendication 12, dans lequel ledit composé de 3a,4,7,7a-tétrahydro-N-(trichlorométhanesulfényl)phtalimide est préparé par
(a) réaction d'un composé organosulfuré avec du chlore dans une solution acide aqueuse pour obtenir du mercaptan méthylique perchloré brut,
(b) isolement de mercaptan méthylique perchloré sensiblement exempt d'impuretés halogénoalcane par traitement du mercaptan méthylique perchloré brut avec un hydrocarbure aromatique, et
(c) réaction du mercaptan méthylique perchloré avec du tétrahydrophtalimide en présence d'un milieu alcalin aqueux pour obtenir un composé de 3a,4,7,7a-tétrahydro-N-(trichlorométhanesulfényl)phtalimide sensiblement exempt d'impuretés halogénoalcane.

15. Procédé selon la revendication 12, dans lequel ledit composé de N-(trichlorométhanesulfényl)phtalimide est préparé par
(a) réaction de disulfure de carbone avec du chlore dans une solution acide aqueuse pour obtenir du mercaptan méthylique perchloré brut,
(b) isolement de mercaptan méthylique perchloré sensiblement exempt d'impuretés halogénoalcane par traitement du mercaptan méthylique perchloré brut obtenu à l'étape (a) avec un hydrocarbure aromatique, et
(c) réaction du mercaptan méthylique perchloré dans l'hydrocarbure aromatique avec un composé de phtalimide dans un milieu alcalin aqueux pour obtenir un composé de N-(trichlorométhanesulfényl)phtalimide sensiblement exempt d'impuretés halogénoalcane.

16. Procédé de préparation de mercaptan méthylique perchloré de formule (II), comprenant
(a) la réaction d'un composé organosulfuré avec du chlore dans une solution acide aqueuse pour obtenir un composé de sulfényle brut, et
(b) le traitement du composé de sulfényle brut avec un hydrocarbure aromatique et l'isolement de mercaptan méthylique perchloré de formule (II) sensiblement exempt d'impuretés halogénoalcane.

17. Procédé selon la revendication 16, comprenant la réaction de disulfure de carbone avec du chlore pour obtenir du mercaptan méthylique perchloré et le traitement du mercaptan méthylique perchloré avec du toluène pour obtenir du mercaptan méthylique perchloré sensiblement exempt d'impuretés halogénoalcane.
